(19) Europäisches Patentamt
European Patent Office
Office européen des brevets

(11) Publication number: **0 022 628**
**B1**

(12) **EUROPEAN PATENT SPECIFICATION**

(45) Date of publication of patent specification: **22.06.83**

(51) Int. Cl.³: **C 07 D 498/04**
//(C07D498/04, 263/00, 205/00)

(21) Application number: **80302054.4**

(22) Date of filing: **18.06.80**

(54) **Process for the preparation of oxazolinoazetidinones.**

(30) Priority: **19.06.79 US 50041**

(43) Date of publication of application:
**21.01.81 Bulletin 81/3**

(45) Publication of the grant of the patent:
**22.06.83 Bulletin 83/25**

(84) Designated Contracting States:
**DE GB LU NL SE**

(56) References cited:
**DD - A - 134 764**
**DE - A - 2 800 860**

(73) Proprietor: **ELI LILLY AND COMPANY**
**307, East McCarty Street**
**Indianapolis Indiana 46285 (US)**

(72) Inventor: **Spitzer, Wayne Alfred**
**5501 Moeller Road**
**Indianapolis Indiana 46524 (US)**
Inventor: **Goodson Jr., Theodore**
**3020 Washington Boulevard**
**Indianapolis Indiana 46205 (US)**

(74) Representative: **Crowther, Terence Roger**
**European Patent Attorney et al,**
**Erl Wood Manor**
**Windlesham Surrey GU20 6PH (GB)**

**EP 0 022 628 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Process for the preparation of oxazolinoazetidinones

This invention relates to a process for the preparation of novel bicyclic intermediates. More particularly the invention is directed to the preparation of certain oxazolinoazetidinones useful in the preparation of oxygen analogues of penicillins.

Recently, in the beta-lactam research area many investigators have directed their attention to the synthesis of noval beta-lactam antibiotics differing from naturally occurring penicillins and cephalosporins not only in the nature of the amino side chain group, or the cephalosporin C—3 group, but also in the nature of the ring-hetero atom (i.e. oxygen, nitrogen or even carbon in place of sulfur). See, for instance, U.S. Patent Specification No. 4,138,486.

There is thus a continuing need for the provision of new intermediates of value in the preparation of such novel $\beta$-lactam derivatives.

Accordingly, the present invention provides a process for preparing an oxazolinoazetidinone of formula (I):

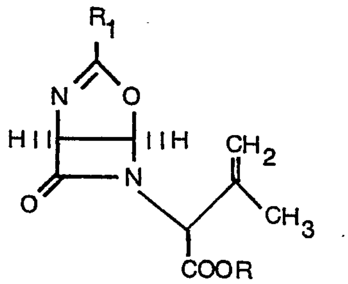

(I)

wherein R is hydrogen or a carboxylic acid protecting group and $R_1$ is a group of the formula

$$R_6(O)_mCH_2—$$

wherein m is 1 or 0 and $R_6$ is phenyl which comprises cyclising a chloroazetidinone compound of formula (II)

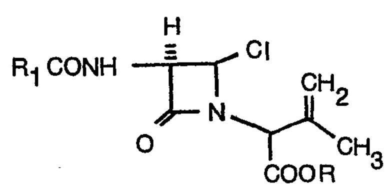

(II)

where R and $R_1$ are as defined above.

The term "carboxylic acid protecting group" has reference to the commonly used carboxylic acid protecting groups employed to block or protect the carboxylic acid functionality while reactions involving other functional sites of the compound are carried out. Such carboxy protecting groups are noted for their ease of cleavage by hydrolytic or by hydrogenolytic methods to the corresponding carboxylic acid. Examples of carboxylic acid ester protecting groups include methyl, *tert*-butyl, benzyl, 4-methoxybenzyl, $C_2$—$C_6$ alkanoyloxymethyl, 2-iodoethyl, 4-nitrobenzyl, diphenylmethyl (benzhydryl), phenacyl, 4-halophenacyl, dimethylallyl, 2,2,2-trichloroethyl, tri($C_1$—$C_3$ alkyl)silyl, succinimidomethyl and like ester forming moieties. In addition to ester protection of carboxy groups, such groups can also be protected as the mixed anhydride, such as that formed with acetyl chloride, propionyl chloride, isobutyryl chloride and like acid chlorides in the presence of a tertiary amine base. Other known carboxy protecting groups such as those described by E. Haslam in *Protective Groups in Organic Chemistry*, supra, Chapter 5, shall be recognized as suitable. The ester forming protecting groups are preferred. The nature of such ester forming groups is not critical.

In the foregoing definitions carboxy protecting groups are not exhaustively defined. The function of such groups is to protect the reactive functional groups during the preparative steps and then to be removed at some later point in time without disrupting the remainder of the molecule. Many protecting groups are known in the art, and the use of other protecting groups not specifically referred to hereinabove are equally applicable.

The $R_1$ groups in accordance with the above definition are benzyl and phenoxymethyl.

As indicated above, the oxazolinoazetidinones of formula (I) can be prepared by cyclising 2-chloroazetidinones of the formula (II)

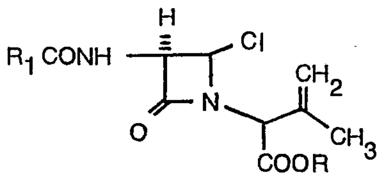

(II)

wherein R and $R_1$ as defined above, the general preparation of which is described in U.S. Patent No. 4,013,653, by an intramolecular nucleophilic displacement reaction. In one method the 2-$\beta$-chloroazetidinone represented by formula (II) can be cyclized to the oxazolinoazetidinones of formula I in almost quantitative yield using lead difluoride in dimethyl sulfoxide. The cyclization reaction is typically conducted at ambient temperature, but can be conducted at temperatures ranging from 0°C to 60°C, preferably from 20° to 30°C.

The reaction mixture is typically heterogenous because lead fluoride has but marginal solubility in dimethyl sulfoxide. The cyclization can be accomplished using from 0.1 to 2.0 molar equivalents of lead difluoride for each molar equivalent of chloroazetidinone starting material. Preferably from 1 to 2 molar equivalents of lead fluoride are employed for each mole of chloroazetidinone. Usually the reaction is conducted by combining equal weights of the chloroazetidinone and lead fluoride in dimethyl sulfoxide at room temperature. At room temperature the reaction is complete in about 1 to 4 hours.

Alternatively, the oxazolinoazetidinones can be prepared from 2-$\beta$-chloroazetidinones of formula (II) by reacting the 2-$\beta$-chloroazetidinone with lithium chloride in acetone to provide a mixture of $\alpha$ and $\beta$-chloroazetidinones and then chromatographing the mixture over silica gel. The $\alpha$-chloroazetidinone cyclizes during chromatography over silica gel. Once formed, the $\alpha$-chloroazetidinone is readily ring-closed to the corresponding oxazolineazetidinone and the reaction may be effected by mild bases such as sodium bicarbonate.

The oxazolineazetidinone compounds produced by the process of the present invention are useful intermediates for the preparation of oxygen analogues of penicillins. Treatment of the compounds of formula (I) with triethylamine in for example ethyl acetate, methylene chloride or chloroform, provides the corresponding $\alpha$,$\beta$-unsaturated oxazolinoazetidinone compounds of formula

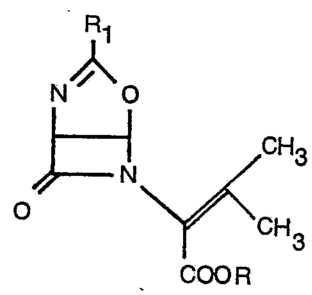

which can be converted to compounds of the formula

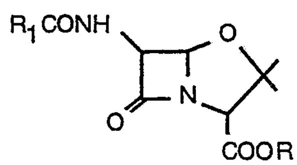

either in accordance with the teaching of U.S. Patents Nos. 3,948,927 and 3,950,352 or in accordance with the teaching of U.S. Patent No. 4,071,512.

Example

p-Nitrobenzyl 2(*R*)-3-methyl-2-[(1*S*,5*R*)-3-phenoxymethyl-7-oxo-2,6-diazabicyclo[3.2.0]-hept-2-en-6-yl]-3-butenoate

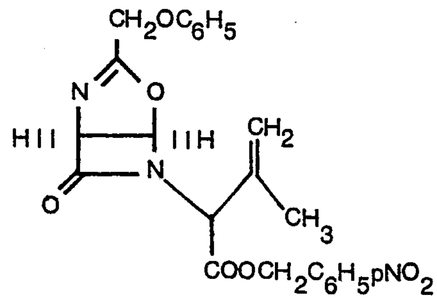

Lead fluoride (3 g) was added to a solution of p-nitrobenzyl 2*R*-(3$\beta$-phenoxyacetamido-2-oxo-4$\beta$-chloroazetidin-1-yl)-3-methyl-3-butenoate (3 g) in dimethyl sulfoxide (30 ml). The suspension was stirred for 4 hours at room temperature. Ethyl acetate (100 ml) was added, and the resulting mixture was washed with saturated sodium chloride solution (3×150 ml).

The organic layer was separated, dried over anhydrous magnesium sulfate and evaporated *in vacuo* to dryness to give a colorless gum. An nmr spectrum of the product showed it to be the title product.

nmr ($CDCl_3$) $\delta$ 1.78 (s, 3, $CH_3$), 4.73 (s, 2, $C_6H_5OC\mathit{H}_2$), 4.9—5.1 (m, 4), 5.26 (s, 2, ester $CH_2$), 6.25 (d, 1, J=3.8 Hz) and 6.83—8.26 (m, 9, ArH).

**Claim**

1. A process for preparing an oxazolinoazetidinone of formula (I):

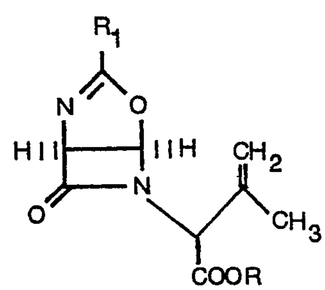
(I)

wherein R is hydrogen or a carboxylic acid protecting group and $R_1$ is a group of the formula

$R_6(OO_mCH_2$—

wherein m is 1 or 0 and $R_6$ is phenyl which comprises cyclising a chloroazetidinone compound of formula (II):

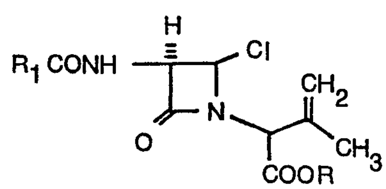
(II)

wherein R and $R_1$ are as defined above.

**Revendication**

Procédé de préparation d'une oxazolinoazétidinone de formule (I):

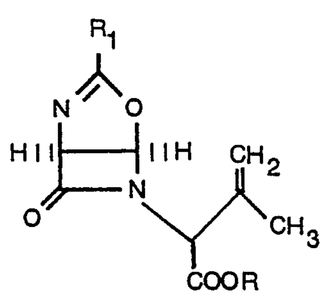
(I)

dans laquelle R représente un atome d'hydrogène ou un groupe protecteur d'acide carboxylique et $R_1$ représente un groupe de formule:

$$R_6(O)_mCH_2—$$

où m est égal à 1 ou 0 et $R_6$ est un groupe phényle, caractérisé en ce qu'il consiste à cycliser un composé de chlorazétidinone de formule (II):

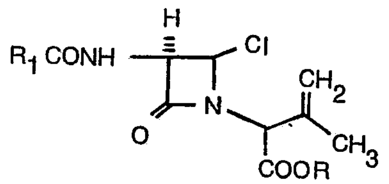

(II)

dans laquelle R et $R_1$ ont les significations définies ci-dessus.

**Patentanspruch**

1. Verfahren zur Herstellung eines Oxazolinoazetidinons der Formel (I)

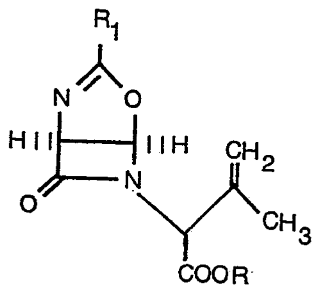

(I)

worin R Wasserstoff oder eine Carbonsäureschutzgruppe ist und $R_1$ eine Gruppe der Formel

$$R_6(O)_mCH_2—$$

bedeutet, worin m für 0 oder 1 steht und $R_6$ Phenyl ist, dadurch gekennzeichnet, daß man eine Chlorazetidinonverbindung der Formel (II)

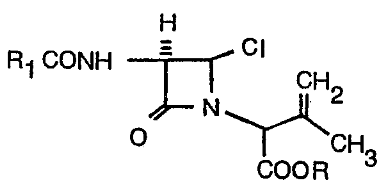

(II)

worin R und $R_1$ die oben angegebenen Bedeutungen haben, cyclisiert.